(19) Europäisches Patentamt
European Patent Office
Office européen des brevets

(11) Veröffentlichungsnummer: **0 024 650**
**B1**

(12) # EUROPÄISCHE PATENTSCHRIFT

(45) Veröffentlichungstag der Patentschrift: **01.12.82**

(21) Anmeldenummer: **80104831.5**

(22) Anmeldetag: **14.08.80**

(51) Int. Cl.³: **C 07 D 265/30**, C 07 D 207/06, C 07 D 207/26, C 07 D 207/32, C 07 D 209/08, C 07 D 211/12, C 07 D 211/14, C 07 D 241/04, C 07 D 277/04 // C09K3/16, C09K3/28

(54) **Di- und/oder Oligomerisierung von organischen, Stickstoffatome enthaltenden heterocyclischen Verbindungen.**

(30) Priorität: **23.08.79 DE 2934131**

(43) Veröffentlichungstag der Anmeldung: **11.03.81 Patentblatt 81/10**

(45) Bekanntmachung des Hinweises auf die Patenterteilung: **01.12.82 Patentblatt 82/48**

(84) Benannte Vertragsstaaten: **BE DE FR GB NL**

(56) Entgegenhaltungen:
**JOURNAL OF THE CHEMICAL SOCIETY, Perkin Transactions I, Nr. 8, 1974, Seiten 891-896 London, G.B. G.D. KHANDELWAL et al.: "Dehydrogenation of Some Aromatic Tertiary Amines by Gamma Radiation and by Peroxides"**

**CHEMICAL ABSTRACTS, Band 93, Nr. 6, 11. August 1980, Seite 1, Nr. 47229s, Columbus, Ohio, U.S.A. H. NAARMANN et al.: "Dimerization and oligomerization by dehydrogenation as a general synthetic principle (1) Part I."**

**CHEMICAL ABSTRACTS, Band 93, Nr. 6, 11. August 1980, Seite 1, Nr. 47230k Columbus, Ohio, U.S.A. H. NAARMANN et al.: "Dimerization and oligomerization by dehydrogenation as a general synthetic principle (1a) Part II."**

(73) Patentinhaber: **BASF Aktiengesellschaft, Carl-Bosch-Strasse 38, D-6700 Ludwigshafen (DE)**

(72) Erfinder: **Naarmann, Herbert, Dr., Haardtblick 15, D-6719 Wattenheim (DE)**
Erfinder: **Zdenek, Janousek, Dr., Université de Louvain, B-1348 Louvain la Neuve (BE)**
Erfinder: **Viehe, Heinz Guenter, Dr., Place L. Pasteur 1, B-1348 Louvain la Neuve (BE)**
Erfinder: **Beaujean, Michel, Place Louis Pasteur 1, B-1348 Louvain la Neuve (BE)**
Erfinder: **Merenyi, Robert, Place L. Pasteur 1, B-1348 Louvain La Neuve (BE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

EP 0 024 650 B1

# Di- und/oder Oligomerisierung von organischen, Stickstoffatome enthaltenden heterocyclischen Verbindungen

Die Erfindung betrifft ein Verfahren zur Di- und/oder Oligomerisierung von organischen, Stickstoffatome enthaltenden heterocyclischen Verbindungen.

Es ist bereits bekannt, durch oxidative Kupplung, z.B. von Isopropylgruppen enthaltenden aromatischen Verbindungen mit Hilfe von Sauerstoff der Peroxiden Di- bzw. Oligomere herzustellen. Derartige Reaktionen sind in den Publikationen von V.V. Korshak et al. in Polymer Science USSR 1 (1962), 925 bis 935 beschrieben. Ähnliche Systeme, aber immer auf Basis von aromatischen Isopropylderivaten, sind in den DE-AS 1244395, DE-AS 1255302, DE-OS 2525697 und DE-OS 2050009 erwähnt. Von L. Friedman und H. Shechter wird im Zusammenhang der Nonylamidmodifizierung von Olefinen die Reaktion von N,N′-Dimethyl- und N-Methylacetamid mit Di-tert.-butylperoxid erwähnt (vgl. Tetrahedron Letters 1961, 238 bis 242).

In J. Chem. Soc., Perkin Transaction I, Nr. 8, (1974) S. 891-896 werden durch Peroxide initiierte Dimerisierungen von N-Aryl-Heterocyclen wie 1-Phenylpyrrolidin beschrieben, wobei über die Eigenschaften und Verwendbarkeit dieser Dimeren nichts ausgesagt ist.

Aufgabe der Erfindung war es, ein Verfahren zur Herstellung von di- und/oder oligomeren Produkten von organischen, Stickstoffatome enthaltenden heterocyclischen Verbindungen aufzufinden.

Diese Aufgabe wurde erfindungsgemäss dadurch gelöst, dass man organische, Stickstoffatome enthaltende heterocyclische Verbindungen der allgemeinen Formeln I oder II:

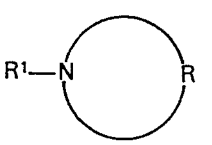 (I)

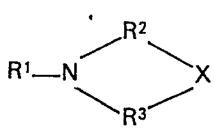 (II)

worin sind:

R = ein $C_4$- bis $C_5$-Atome umfassender, gesättigter oder ungesättigter, gegebenenfalls substituierter Kohlenwasserstoffrest,

$R^1$ = H, $C_1$- bis $C_{10}$-Alkylrest oder Acetylrest,

X = O, NH oder S,

$R^2$ = $CH_2$, gesättigter oder ungesättigter, gegebenenfalls substituierter, 2 C-Atome umfassender Kohlenwasserstoffrest und

$R^3$ = gesättigter oder ungesättigter, gegebenenfalls substituierter, 2 C-Atome umfassender Kohlenwasserstoffrest mit einem organischen Peroxid oder Salcomin im molaren Verhältnis 0,1:1 bis 1:0,1 bei Temperaturen von 120 bis 200°C in Schutzgasatmosphäre zu Verbindungen der allgemeinen Formeln III oder IV:

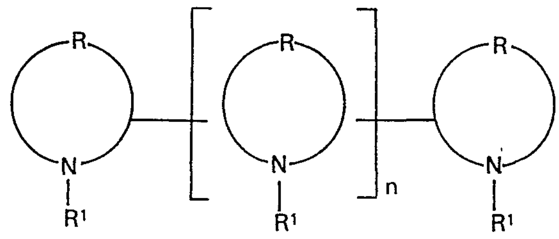

(III) n = 0 bis 10

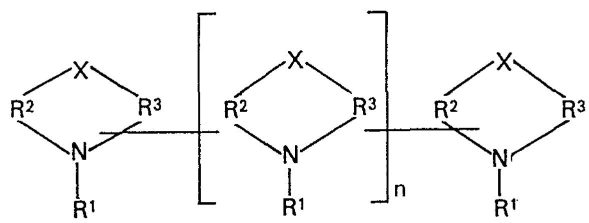

(IV) n = 0 bis 10

umsetzt.

Unter Di- und/oder Oligomerisierung obiger heterocyclischer organischer Stickstoffverbindungen I und II wird eine Reaktion verstanden, bei der zwei oder mehrere, maximal zehn Moleküle der gleichen Verbindung zusammengelagert werden. Derartige Reaktionen sind an sich bekannt und brauchen nicht weiter erläutert werden.

Auch die gemäss der Erfindung einzusetzenden Verbindungen der allgemeinen Formeln I oder II sind an sich bekannt.

Als erfindungsgemässe heterocyclische organische Stickstoffverbindungen der Formeln I und II kommen insbesondere die Verbindungen 1 bis 9 in Betracht:

1. Morpholin
2. N-Acetylmorpholin
3. Piperidin
4. N-Methylpyrrol
5. Pyrrolidin
6. 4-Chlor-N-methylpiperidin
7. 4-Cyan-4-phenylpiperidin
8. Thiazolidin
9. N-Acetylpiperidin-4-carbonsäure

Zur Durchführung der Dimerisierungs- bzw. Oligomerisierungsreaktion werden die heterocyclischen organischen Verbindungen der allgemeinen Formel I oder II mit einem organischen Peroxid, vorzugsweise mit Di-tert-butylperoxid, oder Salcomin im molaren Verhältnis 0,1:1 bis

1:0,1, vorzugsweise im molaren Verhältnis 1:1, bei Temperaturen zwischen 120 und 200°C, vorzugsweise 150 bis 180°C, erhitzt. Als weiteres Peroxid kommt auch Dibenzoylperoxid in Betracht. Als Schutzgasatmosphäre dient ein Inertgas, vorzugsweise Stickstoff. Die erfindungsgemässen Produkte werden durch fraktionierte Destillation, bzw. Umkristallisation, gereinigt.

Die so erhaltenen Di- und/oder Oligomerisierungsprodukte der Verbindungen 1 bzw. 5 weisen die folgenden Strukturformeln auf:

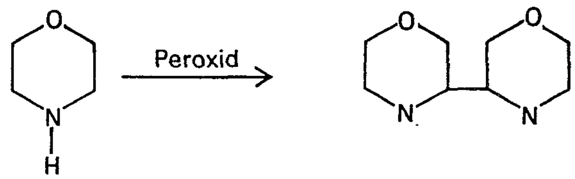


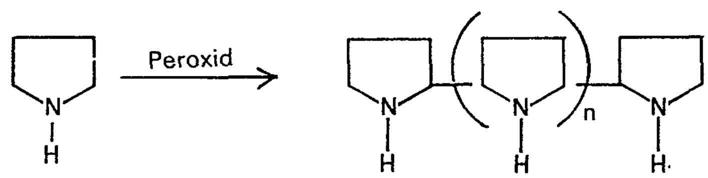


(n = 0 bis 10)

Werden höhere Konzentrationen an Peroxiden eingesetzt, so erfolgt entsprechend dem zweiten Formelschema neben einer Di- auch eine Oligomerisierung.

Die so hergestellten Di- und/oder Oligomerisierungsprodukte können als Antistatika und/oder Flammschutzmittel für Kunststoffe und Fasern verwendet werden.

In den nachfolgenden Beispielen wird die Versuchsdurchführung erläutert. Entsprechend dem Beispiel 1 wurden gemäss der Tabelle weitere N-Heterocyclen umgesetzt und die in der Tabelle angegebenen Dimeren erhalten, wobei die Isolierung entweder als freie Base oder als Salz erfolgte. Die in den Beispielen genannten Teile und Prozente beziehen sich jeweils auf das Gewicht.

Beispiel 1

Dimerisierung von Morpholin

100 Teile Morpholin (Kp 129°C/760 Torr, Molgewicht 87,1) werden mit 10 Teilen Di-tert.-butylperoxid versetzt und sechs Stunden unter Stickstoff auf 160°C erhitzt. Nach dem Abziehen des nicht umgesetzten Morpholins wird das Dimerisierungsprodukt durch fraktionierte Destillation (50 bis 75°C, 10 bis 12 Torr) abgetrennt. Es werden 70 Teile dimeres Morpholin (Molgewicht 190, dampfdruckosmometrisch bestimmt) erhalten.

| Beispiel Nummer | Einsatzstoff | Kp und Fp °C Monomeres | Molgew.-Einsatzstoffe | Ausb. Dimeres in Teilen | Molgew. Dimeres | Kp und Fp °C Dimeres | Bemerkungen zur Aufarbeitung |
|---|---|---|---|---|---|---|---|
| 2 | N-Acetylmorpholin | 59°C Fp 102–105°C/3 Torr | 126 | 74 | 265 | 127–130°C 0,2 Torr | Destillation |
| 3 | Piperidin | 106°C | 85 | 76 | 190 | 202°C Zers. 109°C/2 Torr | Destillation |
| 4 | N-Methylpyrol | 112°C | 67 | 80 | 95 | 84°C/2 Torr | Destillation |
| 5 | Thiazolidin | 72–75°C | 89 | 58 | 210 | 139°C | Umkristallisation Essigester/Pentan |

Beispiel 6

Wird wie im Beispiel 1 beschrieben gearbeitet, jedoch auf 100 Teile Morpholin 25 Teile des Di-tert.-butylperoxids verwendet, so entstehen neben 15 Teilen des dimeren Morpholins ca. 80 Teile Oligomere mit einem «Mittleren Molgewicht» (dampfdruckosmometrisch bestimmt) von 450.

Beispiel 7

Wird wie im Beispiel 1 beschrieben gearbeitet, jedoch das Di-tert.-butylperoxid durch Salcomin ersetzt, so werden 62 Teile des Dimorpholins nach Destillation erhalten.

**Patentanspruch**

Verfahren zur Di- und/oder Oligomerisierung von organischen, Stickstoffatome enthaltenden heterocyclischen Verbindungen, dadurch gekennzeichnet, dass man Verbindungen der allgemeinen Formeln I oder II:

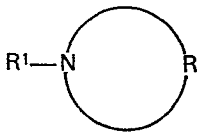

(I)

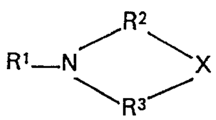

(II)

worin sind:

R = ein $C_4$- bis $C_5$-Atome umfassender, gesättigter oder ungesättigter, gegebenenfalls substituierter Kohlenwasserstoffrest,

$R^1$ = H, $C_1$- bis $C_{10}$-Alkylrest oder Acetylrest,

X = O, NH oder S,

$R^2$ = $CH_2$, gesättigter oder ungesättigter, gegebenenfalls substituierter, 2 C-Atome umfassender Kohlenwasserstoffrest und

$R^3$ = gesättigter oder ungesättigter, gegebenenfalls substituierter, 2 C-Atome umfassender Kohlenwasserstoffrest

mit einem organischen Peroxid oder Salcomin im molaren Verhältnis 0,1:1 bis 1:0,1 bei Temperaturen von 120 bis 200°C in Schutzgasatmosphäre zu Verbindungen der allgemeinen Formeln III oder IV:

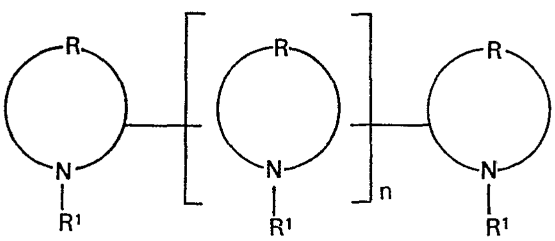

(III) n = 0 bis 10

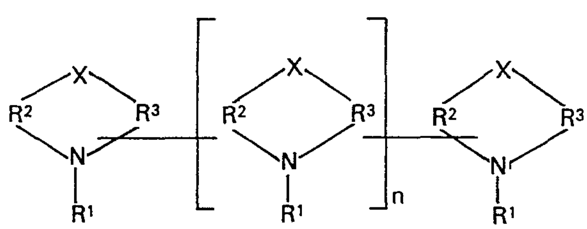

(IV) n = 0 bis 10

umsetzt.

**Claim**

A process for the dimerization and/or oligomerization of organic heterocyclic compounds containing nitrogen atoms, wherein a compound of the general formula I or II

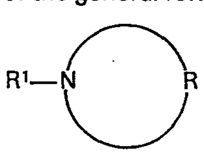

(I)

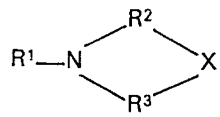

(II)

where R is a saturated or unsaturated, optionally substituted hydrocarbon radical containing 4 or 5 carbon atoms, $R^1$ is H, alkyl of 1 to 10 carbon atoms or acetyl, X is O, NH or S, $R^2$ is $CH_2$, a saturated or unsaturated, optionally substituted hydrocarbon radical containing 2 carbon atoms, and $R^3$ is a saturated or unsaturated, optionally substituted hydrocarbon radical containing 2 carbon atoms, is reacted with an organic peroxide or salcomine in a molar ratio of from 0.1:1 to 1:0.1 at a temperature of from 120° to 200°C in a protective gas atmosphere to give a compound of the general formula III or IV

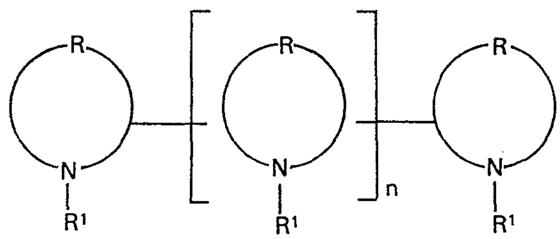

(III) n = 0 to 10

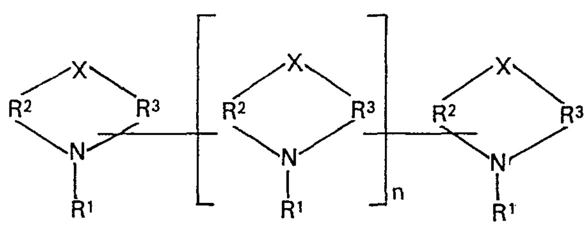

(IV) n = 0 to 10

**Revendication**

Procédé pour la di- et/ou l'oligomérisation de composés organiques hétérocycliques contenant des atomes d'azote, caractérisé en ce que l'on convertit des composés de formules générales I ou II

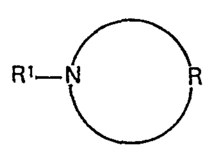

(I)

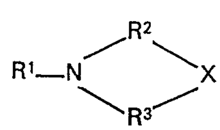

(II)

dans lesquelles

R représente un reste hydrocarboné saturé ou insaturé, éventuellement substitué, en C 4-C 5,

$R^1$ représente H, un groupe alkyle en C 1–C 10 ou acétyle,
X = O, NH ou S,
$R^2$ représente $CH_2$, un reste hydrocarboné saturé ou insaturé, éventuellement substitué, à 2 atomes de carbone et
$R^3$ représente un reste hydrocarboné saturé ou insaturé, éventuellement substitué, à 2 atomes de carbone,
par réaction avec un peroxyde organique ou la salcomine, dans un rapport molaire de 0,1:1 à 1:0,1, à des températures de 120 à 200°C, en atmosphère de gaz protecteur, en composés de formules générales III ou IV

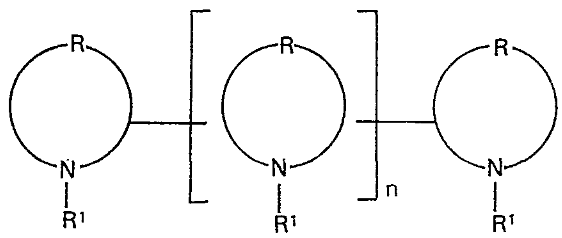

(III) n = 0 à 10

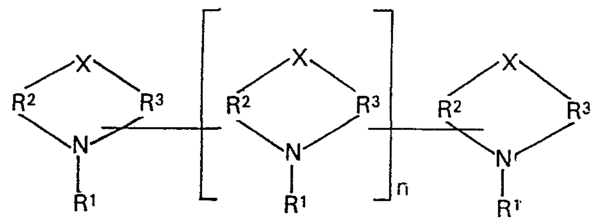

(IV) n = 0 à 10